Europäisches Patentamt

European Patent Office

Office européen des brevets

(19)

(11) Veröffentlichungsnummer : **0 407 990 B1**

(12) ## EUROPÄISCHE PATENTSCHRIFT

(45) Veröffentlichungstag der Patentschrift :
29.09.93 Patentblatt 93/39

(51) Int. Cl.$^5$ : **C07C 19/08**, C07C 17/10

(21) Anmeldenummer : **90113196.1**

(22) Anmeldetag : **11.07.90**

(54) **Verfahren zur Herstellung von 1,1,1-Trifluor-2,2-dichlorethan unter erhöhtem Druck.**

(30) Priorität : **14.07.89 DE 3923248**

(43) Veröffentlichungstag der Anmeldung :
**16.01.91 Patentblatt 91/03**

(45) Bekanntmachung des Hinweises auf die Patenterteilung :
**29.09.93 Patentblatt 93/39**

(84) Benannte Vertragsstaaten :
**AT BE CH DE DK ES FR GB GR IT LI LU NL SE**

(56) Entgegenhaltungen :
**DE-A- 2 755 828
GB-A- 904 831
US-A- 2 861 032
US-A- 4 614 572**

(56) Entgegenhaltungen :
**HOUBEN-WEYL: "METHODEN DER ORGANI-
SCHEN CHEMIE", 4. Auflage, Band V/3, Seiten
594-599, 1962, Georg-Thieme-Verlag, Stuttgart, DE**

(73) Patentinhaber : **HOECHST
AKTIENGESELLSCHAFT
D-65926 Frankfurt (DE)**

(72) Erfinder : **Cremer, Hans Robert, Dr.
Pappelstrasse 1
D-5014 Kerpen (DE)**
Erfinder : **Siegemund, Günter, Dr.
Frankfurter Strasse 21
D-6238 Hofheim am Taunus (DE)**
Erfinder : **Lendle, Wilhelm, Dr.
Hirschpfad 5
D-6232 Bad Soden am Taunus (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 407 990 B1

EP 0 407 990 B1

## Beschreibung

Die Erfindung betrifft ein Verfahren zur Herstellung von 1,1,1-Trifluor-2,2-dichlorethan (R 123) durch Chlorierung von 1,1,1-Trifluor-2-chlorethan (R 133a).

1,1,1-Trifluor-2,2-dichlorethan (R 123) gilt als potentieller Ersatzstoff für den vollhalogenierten Fluorchlorkohlenwasserstoff Fluortrichlormethan (R 11), dessen Produktion und Verbrauch wegen des Verdachts einer Schädigung der Ozonschicht eingeschränkt werden muß. R 123 liefert einen geringeren Beitrag zum Treibhauseffekt und hat aufgrund seines Abbauverhaltens in den unteren Atmosphärenschichten ein erheblich niedrigeres Ozongefährdungspotential. Seine Hauptverwendung findet es als Verschäumungsmittel in der Kunststoffverarbeitung und als Reinigungsmittel.

Man erhält 1,1,1-Trifluor-2,2-dichlorethan ($CF_3$-$CHCl_2$) gemäß US-A-3,755,477 durch Umsetzung von Perchlorethylen mit wasserfreiem Fluorwasserstoff bei Temperaturen von 360°C an Chrom-Katalysatoren. Nachteilig bei diesem Verfahren sind neben der raschen Desaktivierung der Katalysatoren die geringe Ausbeute an $CF_3$-$CHCl_2$ sowie die große Anzahl der wirtschaftlich nicht verwendbaren Nebenprodukte. Zudem ist bei dieser Arbeitsweise nicht auszuschließen, daß sich destillativ nicht abtrennbare, aus toxikologischer Sicht fragwürdige Dichlortrifluorethan-Isomere (z.B. R 123a) bilden.

Eine weitere Darstellungsmethode von 1,1,1-Trifluor-2,2-dichlorethan ist die in der US-A-4,060,469 beschriebene Photochlorierung von 1,1,1-Trifluor-2-chlorethan in der Gasphase. Nachteil dieses Verfahrens ist die sehr geringe Lichtquantenausbeute und der geringe Umsatz. Laut US-A-4,145,368 und US-A-4,192,822 kann die Ausbeute an R 123 zwar erhöht werden, wenn man die Reaktionsprodukte bei 350°C über Chromoxyfluorid-Katalysatoren leitet. Aber auch mit dieser Verfahrensvariante läßt sich die Ausbeute nur auf maximal 14% steigern. Hinzu kommt, daß die verwendeten Cr-O-F-Katalysatoren nicht nur die Dismutierungsreaktion katalysieren, sondern daß auch Dismutierungs-; Isomerisierungs- und Eliminierungsreaktionen an ihnen stattfinden und somit in erheblichem Maße wirtschaftlich nicht verwertbare Nebenprodukte bei dieser Arbeitsweise anfallen.

Weiterhin ist bekannt, daß 1,1,1-Trifluor-2,2-dichlorethan über die thermische Chlorierung von 1,1,1-Trifluorethan zugänglich ist. Wie E.T. McBee et al., Ind. and Engineering Chem., 39, Seiten 409-411 (1947) berichten, wird 1,1,1-Trifluorethan bei 497°C mit der 1,1-molaren Chlormenge umgesetzt (Seite 411 und Tabelle III). Dieses Verfahren ist jedoch für eine industrielle Darstellung von R 123 ungeeignet, da weniger als die Hälfte des eingesetzten Chlors abreagiert und R 123 unter den Reaktionsbedingungen in dem Produktgemisch nur ein Nebenprodukt darstellt. Unter den angegebenen Bedingungen treten neben dem vollhalogenierten Hauptprodukt $CF_3$-$CCl_3$ auch unerwünschte Abbauprodukte (z.B. $CCl_4$, $CF_3Cl$) im Produktspektrum auf.

Es bestand daher die Aufgabe, ein im industriellen Maßstab vorteilhaft durchzuführendes Verfahren zur effizienten und wirtschaftlichen Herstellung von 1,1,1-Trifluor-2,2-dichlorethan bereitzustellen.

Gegenstand der Erfindung ist ein Verfahren zur Herstellung von 1,1,1-Trifluor-2,2-dichlorethan durch Chlorierung von 1,1,1-Trifluor-2-chlorethan, dadurch gekennzeichnet, daß die Chlorierung bei Drücken von 10-400 bar durchgeführt wird.

Unter Normaldruck findet die Chlorierung von 1,1,1-Trifluor-2-chlorethan im allgemeinen erst bei Temperaturen oberhalb 380°C und auch dann nur unvollständig statt. Um so überraschender war die Feststellung, daß durch Anwendung von erhöhtem Druck die Chlorierung von 1,1,1-Trifluor-2-chlorethan schon bei viel tieferen Reaktionstemperaturen mit hohen Umwandlungsraten und hoher Selektivität durchgeführt werden kann.

Bei dem erfindungsgemäßen Verfahren wird 1,1,1-Trifluor-2,2-dichlorethan in isomerenreiner Form erhalten, und es fällt 1,1,1-Trifluor-2,2,2-trichlorethan als praktisch einziges Nebenprodukt an. Hierbei treten somit weder unerwünschte Abbaureaktionen (C-C-Spaltungen) noch andere störende Nebenreaktionen auf.

Das Arbeiten unter Druck nach der vorliegenden Erfindung hat gegenüber der drucklosen Chlorierung den Vorteil, daß neben der höheren Selektivität der Umsatz wesentlich größer ist und sich höhere Raum-Zeit-Ausbeuten erzielen lassen. Das eingesetzte Chlor reagiert vollständig ab und braucht daher nicht anschließend aus dem Produktgemisch abgetrennt zu werden.

Zur Durchführung des Verfahrens werden Drücke von 10 bis 400 bar und insbesondere von 50 bis 250 bar angewandt.

Die Reaktionskomponenten können beim erfindungsgemäßen Verfahren überkritisch oder flüssig vorliegen.

Weiterhin kann die Chlorierung in einem inerten Lösungsmittel, welches die flüssige Phase bildet, durchgeführt werden, wodurch der Druck und die Temperatur natürlich in einem etwas weiteren Bereich als beim lösungsmittelfreien Arbeiten variiert werden kann und sich ein günstiger Verdünnungseffekt erzielen läßt.

Vorteilhafterweise werden in diesem Fall Lösungsmittel eingesetzt, deren kritische Temperaturen oberhalb der angewandten Reaktionstemperaturen liegen und die unter den Chlorierungsbedingungen inert sind. Besonders günstig sind Chlorkohlenwasserstoffe, Fluorkohlenwasserstoffe und Fluorchlorkohlenwasserstof-

2

fe, vorzugsweise Tetrachlormethan und Trichlortrifluorethan.

Das Verfahren kann thermisch oder chemisch eingeleitet (initiiert) werden.

Bei der thermischen Initiierung arbeitet man im allgemeinen bei Temperaturen von 150 bis 400°C, vorzugsweise 200 bis 300°C. Bei der chemischen Initiierung arbeitet man im allgemeinen bei Temperaturen von 100 bis 300°C, vorzugsweise 150 bis 250°C.

Im Falle der chemischen Initiierung finden die für die Chlorierungen bekannten Initiatoren Verwendung. Beispielsweise können hier Diazoverbindungen, wie 2,2'-Azoisobuttersäuredinitril, oder auch Peroxidverbindungen, wie Laurylperoxid und Benzoylperoxid, eingesetzt werden.

Das Verfahren erzielt sehr gute Umsätze bei Initiatormengen von $1 \times 10^{-2}$ bis $1 \times 10^{-6}$, bevorzugt von $1 \times 10^{-3}$ bis $1 \times 10^{-5}$ mol Initiator pro mol Chlor. Höhere Konzentrationen an Initiator (Radikalstarter) sind möglich, aber für einen guten Verfahrensverlauf nicht notwendig.

Das vorliegende Verfahren kann sowohl kontinuierlich als auch diskontinuierlich durchgeführt werden. Bei diskontinuierlicher Reaktionsführung wird das Chlor gasförmig oder flüssig und das 1,1,1-Trifluor-2-chlorethan flüssig, mit oder ohne Lösungsmittel, in den Druckreaktor eingebracht. Im Falle der Verwendung von Radikalstartern können diese direkt in das Druckgefäß zugegeben werden, vorzugsweise werden sie in 1,1,1-Trifluor-2-chlorethan, oder ggf. dem Lösungsmittel gelöst. Der entsprechende Reaktionsdruck ergibt sich aus den Stoffmengen der Einsatzstoffe und der Reaktionstemperatur. Bei kontinuierlicher Arbeitsweise wird der Druck außer durch die Temperatur durch Einpumpen der Reaktionskomponenten erzeugt und durch ein Entspannungsventil konstant gehalten.

Nach Verlassen der Reaktoren werden die Produkte entspannt, der entstehende wasserfreie Chlorwasserstoff abgetrennt und rektifiziert, wobei nicht umgesetztes 1,1,1-Trifluor-2-chlorethan im Kreis geführt und erneut eingesetzt werden kann.

Als Reaktormaterial hat sich Nickel bewährt. Dabei ist Reinnickel zu bevorzugen, jedoch finden auch hochnickelhaltige Stähle und Speziallegierungen als Werkstoffe Verwendung.

Bei der industriellen Anwendung des erfindungsgemäßen Verfahrens sind diverse Reaktorformen möglich. Beispielsweise können Rührkessel, Reaktionskaskaden und Rohrreaktoren eingesetzt werden, vorzugsweise Rohrreaktoren.

Das Molverhältnis Chlor/R 133a sollte im allgemeinen etwa 0,02-1,0 betragen. Wenn hohe Selektivität erwünscht ist, wählt man vorzugsweise ein Molverhältnis von 0,02-0,4. Falls dagegen hoher Umsatz erwünscht ist wählt man vorzugsweise ein Molverhältnis von 0,4-1,0.

In den folgenden Beispielen wird die Erfindung näher erläutert.

Die Produktzusammensetzungen wurden gaschromatographisch analysiert und zusätzlich $^{19}$F- und $^1$H-NMR-spektroskopisch identifiziert. Die angegebenen Prozentangaben sind Gewichtsprozente.

**Beispiel 1**

Ein mit Reinnickel ausgekleideter Autoklav von 1 Liter Inhalt wurde unter Feuchtigkeitsausschluß mit den in Tabelle 1 angegebenen Mengen 1,1,1-Trifluor-2-chlorethan und Chlor beschickt. Mittels einer Mantelheizung wurde der Autoklav auf die gewünschte Reaktionstemperatur gebracht und die Reaktorinnentemperatur während des Versuchs konstant gehalten. Danach wurde auf Raumtemperatur abgekühlt und entspannt. Nach der Chlorwasserstoff-Abtrennung erfolgte die Produktcharakterisierung und die destillative Aufarbeitung.

Die Versuchsresultate sind in Tabelle 1 aufgeführt.

## Tabelle 1

|  |  | Molverhältnis Chlor/R 133a | | |
|---|---|---|---|---|
|  |  | 0,27 | 0,51 | 0,58 |
| Temperatur: | (°C) | 250 | 250 | 250 |
| Druck: | (bar) | 120 | 127 | 122 |
| Reaktionszeit: | (h) | 1 | 1 | 1 |
| Edukte: |  |  |  |  |
| $CF_3-CH_2Cl$ | (g) | 280 | 237 | 225 |
| Chlor | (g) | 45 | 72 | 78 |
| Produkte: |  |  |  |  |
| $CF_3-CH_2Cl$ | (%) | 70,1 | 51,5 | 47,0 |
| $CF_3-CHCl_2$ | (%) | 24,9 | 35,0 | 36,0 |
| $CF_3-CCl_3$ | (%) | 4,7 | 13,5 | 17,0 |
| Andere | (%) | < 0,1 | < 0,1 | < 0,1 |
| Umsatz: |  |  |  |  |
| Chlor | (%) | > 99 | > 99 | > 99 |
| R 133a | (%) | 24,4 | 40,9 | 45,1 |
| Selektivität: |  |  |  |  |
| R 123 | (%) | 83,3 | 72,2 | 67,9 |

**Beispiel 2**

In das im Beispiel 1 beschriebene Druckgefäß wurden 2 mol Tetrachlormethan als inertes Lösungsmittel vorgelegt und die Edukte 1,1,1-Trifluor-2-chlorethan und Chlor zudosiert. Während der Aufheiz- und Reaktionsdauer wurde mittels einer Schüttelvorrichtung die gute Durchmischung der Reaktionsprodukte gewährleistet. Nach Ablauf der jeweiligen Reaktionszeit erfolgte die destillative Aufarbeitung.

In der Tabelle 2 sind die Resultate aufgelistet.

## Tabelle 2

| | | Molverhältnis Chlor/R 133a | |
|---|---|---|---|
| | | 0,26 | 0,24 |
| Temperatur: | (°C) | 200 | 220 |
| Druck: | (bar) | 62 | 70 |
| Reaktionszeit | (h) | 3 | 1 |
| Edukte: | | | |
| $CF_3$-$CH_2Cl$ | (g) | 280 | 280 |
| Chlor | (g) | 43 | 40 |
| Lösungsmittel: | | | |
| $CCl_4$ | (g) | 363 | 363 |
| Produkte: | | | |
| (ohne Tetra) | | | |
| $CF_3$-$CH_2Cl$ | (%) | 70,9 | 73,0 |
| $CF_3$-$CHCl_2$ | (%) | 25,0 | 23,4 |
| $CF_3$-$CCl_3$ | (%) | 4,0 | 3,5 |
| Andere | (%) | < 0,1 | < 0,1 |
| Umsatz: | | | |
| Chlor | (%) | > 99 | > 99 |
| R 133a | (%) | 23,7 | 21,8 |
| Selektivität: | | | |
| R 123 | (%) | 85,9 | 86,7 |

**Beispiel 3**

In 2 mol Tetrachlormethan wurden 100 mg 2,2'-Azoisobuttersäuredinitril gelöst und die Lösung in den in Beispiel 1 beschriebenen Autoklaven eingebracht. Anschließend wurde 1,1,1-Trifluor-2-chlorethan und Chlor zugesetzt und die Reaktion bei 180°C durchgeführt. Nach der Produktaufarbeitung waren die Resultate wie folgt:

## Tabelle 3

| | | Molverhältnis Chlor/R 133a | |
|---|---|---|---|
| | | 0,26 | 0,44 |
| Temperatur: | (°C) | 180 | 180 |
| Druck: | (bar) | 50 | 55 |
| Reaktionszeit: | (h) | 1 | 1 |
| Edukte: | | | |
| CF$_3$-CH$_2$Cl | (g) | 280 | 237 |
| Chlor | (g) | 43 | 64 |
| Radikalstarter: | | | |
| (2,2'-Azoiso- | | | |
| buttersäure- | (mg) | 100 | 100 |
| dinitril) | | | |
| Lösungsmittel: | | | |
| CCl$_4$ | (g) | 363 | 363 |
| Produkte: | | | |
| (ohne Tetra) | | | |
| CF$_3$-CH$_2$Cl | (%) | 71,6 | 53,9 |
| CF$_3$-CHCl$_2$ | (%) | 24,3 | 34,3 |
| CF$_3$-CCl$_3$ | (%) | 4,0 | 11,3 |
| Andere | (%) | < 0,1 | < 0,1 |
| Umsatz: | | | |
| Chlor | (%) | > 99 | > 99 |
| R 133a | (%) | 23,0 | 38,8 |
| Selektivität: | | | |
| R 123 | (%) | 85,6 | 74,4 |

**Patentansprüche**

1. Verfahren zur Herstellung von 1,1,1-Trifluor-2,2-dichlorethan durch Chlorierung von 1,1,1-Trifluor-2-chlorethan, dadurch gekennzeichnet, daß die Chlorierung bei Drücken von 10-400 bar durchgeführt wird.

2. Verfahren nach Anspruch 1, dadurch gekennzeichnet, daß das Reaktionsgemisch überkritisch oder in flüssiger Phase vorliegt.

3. Verfahren nach Anspruch 2, dadurch gekennzeichnet, daß die flüssige Phase durch Druck aufrecht erhalten wird.

4. Verfahren nach Anspruch 2, dadurch gekennzeichnet, daß die flüssige Phase durch ein Lösungsmittel gebildet wird.

5. Verfahren nach Anspruch 4, dadurch gekennzeichnet, daß als Lösungsmittel ein Chlorkohlenwasserstoff, ein Fluorkohlenwasserstoff oder ein Fluorchlorkohlenwasserstoff eingesetzt wird.

6. Verfahren nach Anspruch 5, dadurch gekennzeichnet, daß als Lösungsmittel Tetrachlormethan oder 1,1,1-Trifluor-2,2,2-trichlorethan eingesetzt wird.

7. Verfahren nach einem der Ansprüche 1 bis 6, dadurch gekennzeichnet, daß man bei Drücken von 50 bis 250 bar arbeitet.

8. Verfahren nach einem der Ansprüche 1 bis 7, dadurch gekennzeichnet, daß man die Chlorierung thermisch initiiert und bei Temperaturen von 150 bis 400°C arbeitet.

9. Verfahren nach einem der Ansprüche 1 bis 7, dadurch gekennzeichnet, daß maß die Chlorierung durch Radikalstarter initiiert und bei Temperaturen von 100 bis 300°C arbeitet.

10. Verfahren nach einem der Ansprüche 1 bis 7, dadurch gekennzeichnet, daß man die Chlorierung durch Radikalstarter initiiert und bei 150 bis 250°C arbeitet.

11. Verfahren nach Anspruch 9 oder 10, dadurch gekennzeichnet, daß als Radikalstarter eine Diazoverbindung oder eine Peroxidverbindung verwendet wird.

12. Verfahren nach Anspruch 9 oder 10, dadurch gekennzeichnet, daß als Radikalstarter 2,2'-Azoisobuttersäuredinitril verwendet wird.

13. Verfahren nach einem der Ansprüche 1 bis 12, dadurch gekennzeichnet, daß das Molverhältnis Chlor/1,1,1-Trichlor-2-chlorethan zwischen 0,02 und 1,0 liegt.

14. Verfahren nach einem der Ansprüche 1 bis 13, dadurch gekennzeichnet, daß die Umsetzung kontinuierlich durchgeführt wird.

15. Verfahren nach einem der Ansprüche 1 bis 14, dadurch gekennzeichnet, daß die Umsetzung in einem Rohrreaktor durchgeführt wird.

## Claims

1. A process for the preparation of 1,1,1-trifluoro-2,2-dichloroethane by chlorination of 1,1,1-trifluoro-2-chloroethane, which comprises carrying out the chlorination at pressures of 10-400 bar.

2. Process as claimed in claim 1, wherein the reaction mixture is present in a supercritical state or in the liquid state.

3. The process as claimed in claim 2, wherein the liquid phase is maintained by means of pressure.

4. The process as claimed in claim 2, wherein the liquid phase is formed by a solvent.

5. The process as claimed in claim 4, wherein the solvent employed is a chlorinated hydrocarbon, a fluorinated hydrocarbon or fluorinated and chlorinated hydrocarbon

6. The process as claimed in claim 5, wherein the solvent employed is carbon tetrachloride or 1,1,1-trifluoro-2,2,2-trichloroethane.

7. The process as claimed in any one of claims 1 to 6, wherein the reaction is carried out at pressures of 50 to 250 bar.

8. The process as claimed in any one of claims 1 to 7, wherein the chlorination is initiated thermally and is carried out at temperatures from 150 to 400°C.

9. The process as claimed in any one of claims 1 to 7, wherein the chlorination is initiated by free-radical initiators and is carried out at temperatures from 100 to 300°C.

10. The process as claimed in any one of claims 1 to 7, wherein the chlorination is initiated by free-radical initiators and is carried out at 150 to 250°C.

11. The process as claimed in claim 9 or 10, wherein the free-radical initiator used is a diazo compound or a peroxide compound.

12. The process as claimed in claim 9 or 10, wherein 2,2'-azoisobutyrodinitrile is used as the free-radical initiator.

13. The process as claimed in any one of claims 1 to 12, wherein the chlorine/1,1,1-trichloro-2-chloroethane molar ratio is between 0.02 and 1.0.

14. The process as claimed in any one of claims 1 to 13, wherein the reaction is carried out continuously.

15. The process as claimed in any one of claims 1 to 14, wherein the reaction is carried out in a tubular reactor.

## Revendications

1. Procédé pour préparer du 1,1,1-trifluoro-2,2-dichloréthane par chloration du 1,1,1-trifluoro-2-chloréthane, caractérisé en ce qu'on effectue la chloration sous des pressions de 10 à 400 bars.

2. Procédé selon la revendication 1, caractérisé en ce que le mélange réactionnel est présent dans un état surcritique ou en phase liquide.

3. Procédé selon la revendication 2, caractérisé en ce qu'on maintient la phase liquide sous l'effet de la pression.

4. Procédé selon la revendication 2, caractérisé en ce que la phase liquide est obtenue à l'aide d'un solvant.

5. Procédé selon la revendication 4, caractérisé en ce qu'on utilise comme solvant un hydrocarbure chloré, un hydrocarbure fluoré ou un hydrocarbure fluorochloré.

6. Procédé selon la revendication 5, caractérisé en ce qu'on utilise comme solvant le tétrachlorométhane ou le 1,1,1-trifluoro-2,2,2-trichloréthane.

7. Procédé selon l'une des revendications 1 à 6, caractérisé en ce qu'on travaille sous des pressions de 50 à 250 bars.

8. Procédé selon l'une des revendications 1 à 7, caractérisé en ce qu'on amorce thermiquement la chloration et l'on travaille à des températures de 150 à 400°C.

9. Procédé selon l'une des revendications 1 à 7, caractérisé en ce qu'on amorce la chloration par des amorceurs générateurs de radicaux libres, et l'on travaille à des températures de 100 à 300°C.

10. Procédé selon l'une des revendications 1 à 7, caractérisé en ce qu'on amorce la chloration à l'aide d'amorceurs générateurs de radicaux libres, et l'on travaille entre 150 et 250°C.

11. Procédé selon la revendication 9 ou 10, caractérisé en ce qu'on utilise comme amorceur générateur de radicaux libres un composé diazoïque ou un peroxyde.

12. Procédé selon la revendication 9 ou 10, caractérisé en ce qu'on utilise comme amorceur générateur de radicaux libres le 2,2'-azoisobutyrodinitrile.

13. Procédé selon l'une des revendications 1 à 12, caractérisé en ce que le rapport molaire entre le chlore et le 1,1,1-trichloro-2-chloréthane se situe entre 0,02 et 1,0.

14. Procédé selon l'une des revendications 1 à 13, caractérisé en ce qu'on conduit la réaction en continu.

15. Procédé selon l'une des revendications 1 à 14 caractérisé en ce qu'on effectue la réaction dans un réacteur tubulaire.